# EUROPEAN PATENT APPLICATION

(11) **EP 1 266 964 A1**
(43) Date of publication of application: **18.12.2002**
(21) Application number: 01908354.2
(22) Date of filing: 06.03.2001
(51) Int. Cl.: C12N 15/12, C07K 14/435, C12P 21/02, C08G 85/00, D01F 4/00

(54) **PRECURSORS OF SILK-LIKE MATERIALS, SILK-LIKE MATERIALS AND PROCESSES FOR PRODUCING BOTH**

(30) Priority: 24.03.2000 JP 2000084141
(71) Applicant: Japan as represented by President of Tokyo University of Agriculture and Technology, Fuchu-shi, Tokyo 183-8583 (JP)
(72) Inventor: ASAKURA, Tetsuro, Tokyo University, Koganei-shi, Tokyo 184-0012 (JP)
(74) Representative: Wilson Gunn Gee
(86) International application number: JP0101733
(87) International publication number: WO01070973

(57) **Abstract**

A silk-like material precursor, a method of producing such a precursor and a silk-like material produced from such a precursor are provided.

The silk-like material precursor is a copolymer represented by the general formula -[(GA¹)ⱼ-((GA²)ₖ-G-Y-(GA³)ₗ)ₘ]ₙ-. In the module (GA¹)ⱼ, the precursor is organized in a repeated β-turn type II structure having intra-molecular hydrogen bonds formed successively along its molecular axis. In this formula, G represents glycine and A¹ represents alanine, but it is also possible to replace every third A¹ with serine. A² and A³ both represent alanine, but these residues may be partly replaced with valine. Y represents an amino acid containing an asymmetric carbon atom giving water solubility to the precursor. Finally, j is an integer of at least 6, k and l are both integers of 0 to 5, m is an integer of 1 to 7 and n is an integer of at least 10.

## Description

### Technical Field

The present invention relates to precursors of silk-like material, a silk-like material produced therefrom, and methods of producing the same.

### Background of the Invention

Domestic silkworms (*Bombyx mori* silkworms) extrude liquid silk in a pre-fibrillation state and produce silk fibers with high strength and high elasticity within a very short time of spinning at room temperature. It is well known that fabric woven from such silk fibers has widely been praised for luxury clothing production. In addition, this kind of textile fabric is ecologically friendly since silk fibers are biodegradable. Therefore, in the past decades, natural silk has become the subject of extensive studies as model systems. Although the primary structure of *Bombyx mori* silk and the secondary structure of silk fibers (Silk II) have already been determined, the structure of silk before spinning (Silk I) and the conformational transition involved have not been clarified yet. Moreover, many attempts to produce such silk-like fibers with high strength and elasticity have been performed but none of them really succeeded.

The primary structure of the silk fibroin produced by domestic silkworms has been reported by Zhou et al. (Zhou et al., Nucleic Acids Research, **28**, 2413-2419 (2000)). According to this report, the crystalline repeated motifs (Gly-Ala-Gly-Ala- Gly-Ser)n and the semicrystalline motifs containing Tyr and/or Val are partly included in a chain of Gly-Ala linkage. Furthermore, both the ordered and the disordered domains are repeated in cycles. Besides, the structure of silk films (liquid silk extracted from the posterior silk glands and gently dried) has been studied on the atomic level by X-ray diffraction. In this case, the structure reported is namely Silk I. Synthesis and structural analysis of several model compounds of silk have also been performed. However, the Silk I structure still remain poorly characterized. Therefore, the precise mechanism of formation of silkworm silk fibers cannot be determined. Without this fundamental knowledge, it is undoubtedly difficult to produce silk-like fibers artificially.

Previous models of the Silk I structure include the well known "Crankshaft Model" proposed by Lotz et al. and the "Out-of-Register Model" proposed by Fossey et al. The former model is based on X-ray data analysis, electron-beam analysis and conformational energy calculations. The latter model is only based on elaborate conformational energy calculations. However, these two models cannot explain all the X-ray data. Moreover, the conformational transition from Silk I to Silk II structures requires the cleavage of inter-molecular hydrogen bonds and further, the formation of other hydrogen bonds. These requirements are not fulfilled either for the "Crankshaft" or the "Out-of-Register Model" models since in these cases, all the hydrogen bonds are inter-molecular.

The present researcher has performed intensive studies on Silk I structure by using new analytical methods based on solid-state nuclear magnetic resonance (NMR). This researcher has also succeeded in determining the Silk I structure precisely and the following invention has been achieved.

The first object of the invention is to provide the precursor of silk-like material, that is, the liquid silk.

The second object of the invention is to provide the method for producing the precursor of silk-like material.

The third object of the invention is to provide silk-like materials that can be synthesized from the precursor.

The fourth object of the invention is to provide a method of producing silk-like materials including silk.

### Disclosure of the Invention

The silk-like material precursor is designed as a copolymer (precursor) represented by the general formula - [(GA¹)ⱼ-((GA²)ₖ-G-Y-(GA³)ₗ)ₘ]ₙ. The precursor is organized in repeated β-turn type II structure wherein intra-molecular and inter-molecular hydrogen bonds are formed alternatively along the molecular axis. The whole invention proposes a method of producing such a precursor, a liquid silk from this precursor and then silk-like fibers spun out from the silk solution.

### Brief Description of the Pictures

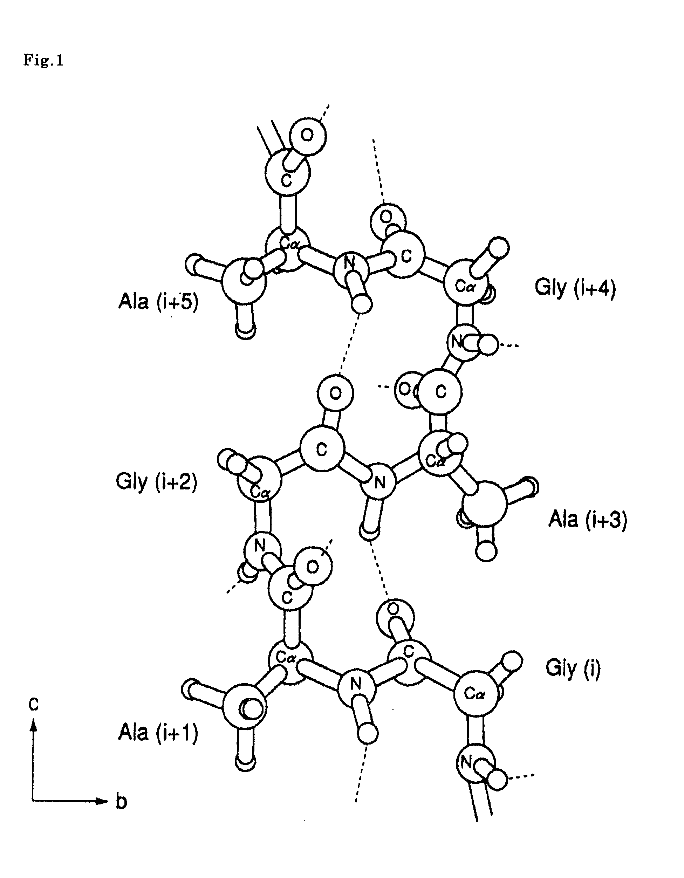

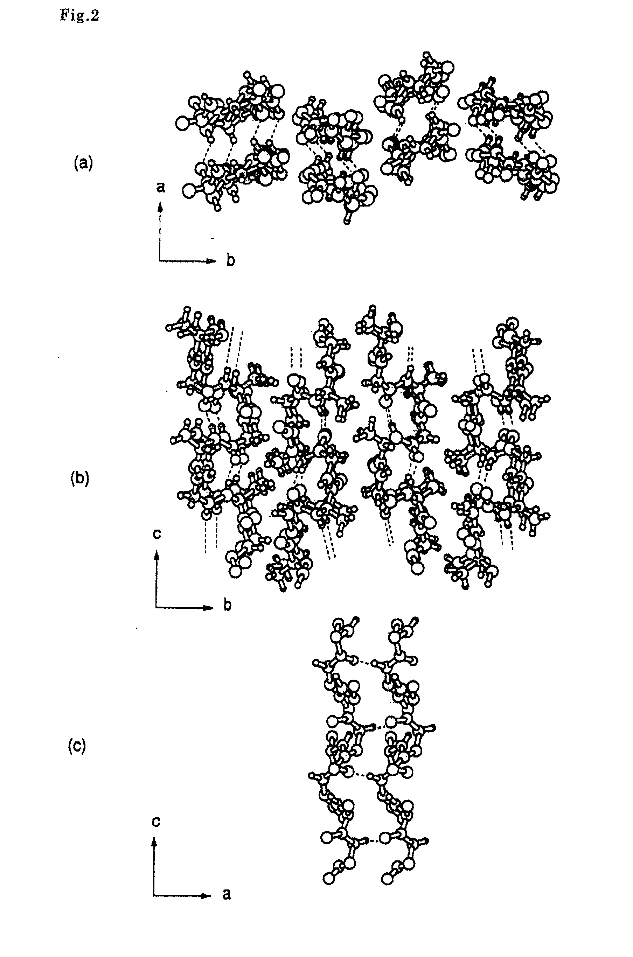

In Fig. 1 is depicted the Silk I structure of the silk-like precursor. Each broken line represents intra-molecular hydrogen bonds. The pictures presented in (a), (b) and (c) of Fig.2 show inter-molecular hydrogen bonds formed by the silk-like material precursor having the Silk I structure. Additionally, the broken lines in the Fig.2b represent intra-molecular hydrogen bonds and those in the Fig.2a and (c) stand for inter-molecular hydrogen bonds.

### Best Mode for Carrying Out the Invention

The formula -[(GA¹)ⱼ-((GA²)ₖ-G-Y-(GA³)ₗ)ₘ]ₙ- representing the silk-like material precursor is not necessarily applied to the chain ends. In the above formula, G represents glycine and Y represents an amino acid having an asymmetric carbon atom, allowing water solubility to the precursor. Examples of such an amino acid include L-glutamic acid, L-lysine and L-tyrosine, the latter being particularly suitable. Furthermore, "L-" stands for a levo-rotatory optical isomer. A¹ represents alanine but it is also possible to replace every third alanine with serine. A² and A³ both represent alanine, but they may be partly replaced with valines.

The formula -[(GA¹)ⱼ-((GA²)ₖ-G-Y-(GA³)ₗ)ₘ]ₙ is represented in a block of copolymer form. The present precursor is not confined to those having a block of copolymer form though. For the subscript letters, j is an integer of at least 6, k and l are both integers of 0 to 5, m is an integer of 1 to 7 and n is an integer of at least 10. Additionally, Y may include different kinds of amino acids, but it is especially advantageous that Y represents L-tyrosine alone. The water solubility of the precursor varies depending on the content of Y and it is appropriate that the content of Y is 20 mole % or below. Although n is an integer of at least 10 as defined above, it is desirable for the spinning point of view capability that n is a value enabling the copolymer to have a molecular weight of at least 30,000.

The term "repeated β-turn type II structure" used in the description of the invention represents a structure in which, as illustrated in Fig. 1, the amino acid residues are arranged in repeated turns having a helical fashion. In contrast to the Silk II structure and to the traditional models, such repeated β-turn type II structure enables the precursor, represented by the formula mentioned above, to sequentially form intra-molecular hydrogen bonds along its molecular axis. These intra-molecular hydrogen bonds are represented by broken lines in Fig.1 and Fig.2(b). A method of producing the present silk-like material precursor is described in details below.

Before to produce the precursor, the amounts of amino acids to be used together with glycine are first specified. Furthermore, the amount of Y is determined depending on the desired water solubility. According to the total amino acids composition, it is appropriate that the proportion of Y is 20 mole % or below. When the proportion of Y is increased beyond 20 mole %, the stability of the precursor decreases and prevents the fibrillation to occur. Moreover, it is required that Y is introduced in the main chain. The hardness of the copolymer can also be controlled by introducing a serine residue in every third position of a sequence of alanines represented by A¹. The appropriate proportion of serine has to be considered as well.

The precursor can be synthesized using a solid-phase peptide synthesis method or a genetic engineering method using bacteria (*Escherichia coli*). When the latter method is adopted to produce a copolymer having a high molecular weight, the general formula of the precursor is determined first. Then, the sequence of oligonucleotides into which the desired sequence is encoded is synthesized chemically. By using these oligonucleotides, the repetitive unit of the general formula is produced. If it is necessary, this sequence is incorporated into a cloning vector and then transferred into *Escherichia coli.* The last step will allow the polymerization of the precursor to an appropriate extend. In the present invention, it is advantageous to adopt the genetic engineering method characterized by the aforementioned steps. An analogous method is also enclosed in the Prince J,T et al. work (Biochemistry 34, 10879 (1995)).

Fig.1 is a picture presenting the Silk I structure of a copolymer of glycine and alanine, wherein each intra-molecular hydrogen bond is formed along the molecular axis. The pictures in Fig.2 demonstrate a regular inter-molecular structure. More specifically, the pictures (a), (b) and (c) are c-axis projection, a-axis projection and b-axis projection, respectively. In addition to the well-ordered structure represented by the Silk I structure, the precursor may contain amorphous regions as well.

In a molecule adopting the Silk I structure, as shown in Fig.1 and Fig.2(b), three amino acid residues form an intra-molecular hydrogen bond between the amino group located at one end (e.g., Ala(i+3)) and the oxygen atom of the carbonyl group positioned at the other end (e.g., Gly(i)). By forming such intra-molecular hydrogen bond, the present copolymer can assume a cyclic structure referred to the "β-turn type II structure". As shown in Fig.1, the two peptide groups participating in the intra-molecular hydrogen bonding of the β-turn type II structure are also shared by other chain with β-turn type II structure.

As shown in Fig.2(a) and Fig.2(c), when the molecules organized in the so-called Silk I structure are in the solid state, the adjacent molecules along the a-axis direction are bounded by inter-molecular hydrogen interactions. As depicted in Fig.1, each inter-molecular hydrogen bond is formed between amino acid residues whose are not participating to intra-molecular hydrogen bonding. More specifically, the Silk I solid-state structure is composed of amino acid residues participating in intra-molecular hydrogen bonding and amino acid residues contributing to inter-molecular hydrogen bonding, which alternate with each other.

The formation of intra-molecular hydrogen bonds in Silk I structure can be confirmed by determining the distance between the carbon and the nitrogen atoms participating in the intra-molecular bonding by using a recent solid-state NMR method named REDOR. This technique is very useful to determine accurately the distance between two atoms labeled (e.g. ¹³C and ¹⁵N).

Overall, as described below, the repeated β-turn type II model gives a reasonable explanation for the mechanism of silk fiber formation from liquid silk extruded by domestic silkworms.

The present precursor of Silk I structure has a high thermal stability in the solid state. However, by stretching along the fiber axis direction, the Silk I structure changes easily to Silk II structure. Such a transition can be understood looking at the intra-molecular hydrogen bonds whose are approximately parallel to the molecular axis. Moreover, this conformational transition can also be understood in terms of close packing of the individual chains, as shown in Fig.1 and Fig.2(b). As we can observe in the Silk I structure, the intra-molecular hydrogen bonds are almost parallel to the molecular axis and so these bonds are easily broken upon stretching along this axis. Then, the inter-molecular hydrogen bonds are newly formed perpendicularly to the molecular axis. The individual sets of amino acids participating in the intra-molecular hydrogen bonds of each molecule stands in proximity and are facing the residues involved in intra-molecular hydrogen bonds present in adjacent molecules. Consequently, the formation of inter-molecular hydrogen bonds occurs simultaneously with the cleavage of intra-molecular hydrogen bonds in the Silk I structure. This passage can be referred as the "transition of intra-molecular hydrogen bonds".

In the present invention, a copolymer is synthesized in *Escherichia coil*, purified and dissolved in LiBr aqueous solution. The LiBr is removed from the solution by dialysis and the water is then gently evaporated. Thereby, the precursor is obtained. In the process of removing water, a silk film is usually obtained. At this step, when the initial aqueous solution has a low silk concentration, the precursor can be amorphous. It is also possible to let the present precursor adopt the Silk I structure by having an initial aqueous solution with a higher silk concentration. The Silk I structure obtained can also be converted into the Silk II structure by stretching the film or treat it with formic acid. However, in order to produce silk fibers, it is required to spin out the silk from the aqueous solution before the film formation occurs. It is also possible to produce silk fibers by dissolving the precursor obtained as a film, e.g., in hexafluoroisopropanol (HFI) or so, and then spin out the fibers from the resulting solution. The spinning conditions may be determined appropriately according to the physical properties of the precursor obtained. Furthermore, by selecting the orifice opening of a spinning nozzle, it is possible to produce silk-like fibers having various diameters. Basically, for low production costs and eco-friendliness, it is preferable to avoid the use of a solvent such as hexafluoroisopropanol. It is certainly more appropriate to spin out the silk-like precursor from an aqueous solution. However, it is also possible to design the precursor such that it can be dissolved in organic solvents such as alcohol or ether.

In general, several functions can be added to the silk-like material, prepared either as a film or as a fiber, by modifying the composition of the precursor. Moreover, the precursor is a protein and is degradable upon the action of microbes. Besides, it has the unique properties of natural silk and its method of production is ecological.

The present invention will be illustrated in more details with a few examples. It is important to stress the fact that these examples should not be considered as limiting for the scope of the present invention.

### EXAMPLES

### EXAMPLE 1:

Comparative examinations of conformational transitions of the tyrosine residues present in silk were made. The silk samples were prepared from domestic silkworms and were isotopically labeled in ¹³C for the L-tyrosine residues. To do so, 50 mg of ¹³C-labeled L-tyrosine was given to silkworm larvae in the fifth instar stage. The silk samples were then characterized by solid-state high-resolution ¹³C NMR experiments. It was confirmed that the L-tyrosines took random coil conformations in both liquid silk and film samples formed by drying. However, these residues were rather organized in Silk II structures when the silk was spun out to form the cocoons. Silk fibers prepared by dissolving the silk films in hexafluoroisopropanol and spun out from the resulting solution were also studied by orientated-solid state NMR measurements. It was clear that L-tyrosine were involved in Silk II structures. In each molecular chain, these residues were also well orientated. In addition, it was confirmed by solid-state NMR measurements that both alanine and glycine residues, whose are the main constituents of silk, took the Silk I structure either in the liquid silk and or in the dried film samples but they were rather involved in the Silk II structure in the silk fibers.

### EXAMPLE 2:

The following peptides I, II, III and IV, whose are representative chain segments in the repeated domains of *Bombyx mori* silk, were synthesized using solid-phase methods. The solubility in water was also examined. The model compound I was insoluble in water but the peptides II, III and IV were highly soluble. By these examinations, it was therefore verified that tyrosine residues are essential for giving solubility to silk in water.

### Synthesized Compounds:

G(AG)₂SG(AG)₂ I

G(AG)₂YG(AG)₂ II

GAGVGYG(AG)₂ III

TGFDSESSWAYEYGSYGGNAVYPGFGSSGT IV

Herein, G stands for glycine, A for alanine, Y for tyrosine, V for valine, T for threonine, F for phenylalanine, D for aspartic acid, E for glutamic acid, W for tryptophan, N for asparagine and P for proline. All these amino acids are L-amino acids excepted glycine.

### EXAMPLE 3:

The following model compounds of silk were synthesized using solid-phase methods.

G(AG)₂YG(GA)₂ II

(AG)₆YG(AG)₅ V

(AG)₇YG(AG)₇ VI

(AG)₁₅ VII

Herein, A stands for L-alanine, G for glycine and Y for L-tyrosine.

As mentioned in the Example 2, the sample II was highly soluble in water. The Sample V was studied as a gel with solid-state high-resolution ¹³C NMR. In this case, the Silk I structure was adopted. The gel was prepared by dissolving 300 mg of Sample V in 7 ml of a 9M LiBr aqueous solution. In order to remove the LiBr, the solution was then dialyzed for 3 days and the sample had a gel appearance in the membrane used for the dialysis. Through the same procedure mentioned above, a gelatin texture was also observed for the Sample VI. However, both structures Silk I and Silk II were present in this sample. Finally, for the sample VII, a precipitate was formed in the membrane for dialysis. This precipitate was found to adopt the Silk I structure.

### EXAMPLE 4:

The following model compounds of silk were synthesized using solid-phase methods.

(AG)₃YG(AG)₃YG(AG)₃YG(AG)₃ VIII

(AG)₃YG(AG)₂VGYG(AG)₃YG(AG)₃ IX

(AGAGYG)₅ X

An amount of 300 mg of the samples VIII, IX and X was dissolved in 7 ml of a 9M LiBr aqueous solution. The solution was then dialyzed for 3 days to remove the LiBr. During the dialysis process, all peptides took a gel-like form in the membrane for dialysis. As suited, the resulting materials presented high water contents. During the dialysis process, the gel-like form was clearly different from the one observed for the sample VII ((AG)₁₅). Thus the tyrosine residues introduced in each copolymer had proved to give a hydrophilic character to the copolymer.

### EXAMPLE 5:

In order to prepare an aqueous solution of silk fibroin, 1 g of degummed fibers (without sericin) were dissolved in 50 ml of a 9M LiBr aqueous solution and kept at 40°C for 1.5 hours. The insoluble components were filtered from the solution. An aqueous solution of 9M LiBr with a silk concentration of 0.5 % was obtained. The solution was then transferred into a membrane and dialyzed against distilled water for 4 days. The aqueous solution with a silk concentration lower than 0.5 % was prepared as well. The silk solution was spread on No.2 square Petri dishes and dried moderately at room temperature. An amount of 0.15 g per sheet was obtained from the resulting amorphous films. These films were soluble in HFIP at room temperature (15°C to 30°C), and it took about 3.5 hours for a complete dissolution of each film in the solvent. The fibers spun out from the HFIP solution were drawn and their structure was characterized. As a result, it was confirmed that the drawn fibers took the Silk II structure.

### EXAMPLE 6:

The following model compounds of silk were synthesized using solid-phase methods.

(AG)₆A[1-¹³C]G[1-¹³C]AG(AG)₇ XI

(AG)₇[1-¹³C]A[1-¹³C]G(AG)₇ XII

(AG)₆A[1-¹³C]GA[1-¹³C]G(AG)₇ XIII

(AG)₆A[1-¹³C]GA[¹⁵N]G(AG)₇ XIV

(AG)₇[1-¹³C]AG[¹⁵N]AG(AG) 6₇ XV

An amount of 300 mg of each compound was dissolved in 7 ml of a 9M LiBr aqueous solution and dialyzed for 3 days leading to the formation of a precipitate in the dialysis membrane. IR spectra indicated that the precipitate adopts Silk I structure. Two-dimensional ¹³C solid-state NMR spin diffusion experiments were performed on the model compounds XI, XII and XIII. REDOR NMR measurements were also used to characterize the samples XIV and XV. The symbols [1-¹³C]G and [1-¹³C]A represent glycine and alanine residues in which the carbon of the carbonyl group is labeled with ¹³C, respectively. The symbols [¹⁵N]G and [¹⁵N]A represent glycine and alanine residues labeled with ¹⁵N, respectively.

Based on spectral analysis, the torsion angles (φ,ϕ) of the alanine residues included in the model compound XI were determined and are the following: -60°±5° and 130°±5°, respectively. The torsion angles (φ,ϕ) of the glycine residues included in the model compounds XII and XIII were also determined and are the following: 70°±5° and 30°±5°, respectively. By REDOR measurements, it has also been found that the torsion angles (φ,ϕ) are the same for the alanine and glycine reissued incorporated in the compounds XIV and XV.

Finally, when the torsion angles of a copolymer composed by alternating alanine and glycine residues were adjusted to the values determined above, the copolymer adopted the structure shown in Fig.1 (Silk I).

### EXAMPLE 7:

The following model compounds of silk were synthesized using solid-phase methods.

(AG)₁₅ VII

(AG)₆A[1-¹³C]GAG[¹⁵N]AG(AG)₆ XVI

As presented in the Example 1, these samples were prepared such that the Silk I structure is formed. By REDOR measurements of the sample XVI, the distance between the [1-¹³C]G₁₄ residue and the [¹⁵N]A₁₇ residue was determined to be 4.0±0.1 Å. Such a distance was also measured for an equal mixture of samples XVI and VII. Therefore, it seems that there is no inter-molecular influence on the determination of atomic distances. Moreover, the distance 4.0±0.1 Å is in good agreement with the distance between the corresponding atoms in the structure depicted in Fig.1. This fact proves clearly that Silk I structure forms intra-molecular hydrogen bonding.

### EXAMPLE 8:

Degummed silk fibroin fibers (1 g) were added to a 9M LiBr aqueous solution (50 ml) and kept at 40°C for 1.5 hours. The resulting solution was filtered in order to remove the insoluble components. An aqueous solution of 9M LiBr with a silk concentration of 0.5 % was obtained and transferred into a membrane and dialyzed against distilled water for 4 days. The aqueous solution with a silk concentration lower than 0.5 % was prepared as well. The pH of the silk solution (containing 4 g of silk) was adjusted to 7.8 by addition of sodium phosphate. Then, several ml of an aqueous solution containing chymotrypsin was added to the solution and followed by a 24-hours incubation at 40°C. The precipitate was centrifuged at 10,000 r.p.m. and the enzymatic reaction was ended by the addition of 0.03 N hydrochloric acid. The precipitate was then washed and a CP fraction of about 55 % of the original silk fibroin was obtained. The sequence of the CP fraction has been previously characterized and is mainly composed by repetitive motifs of AGAGSG (Strydom et al., Biochem. Biophys. Res. Commun., 3, 932 (1977)).

The CP fraction we obtained was dissolved in an aqueous solution of 9M LiBr and dialyzed. By IR measurements, we observed that the sample was adopting the so-called Silk I structure. ¹³C solid-state high-resolution NMR spectra of this CP fraction and the model compound VII ((AG)₁₅) were acquired. As a result, it was found that the peaks position and the line shapes arising from the alanine and glycine residues were the same for these two samples. Thus, it can be concluded that the present alanine-glycine copolymer is a representative model of the crystalline domain (AGAGSG)n of *Bombyx mori* silk. By treating the samples in formic acid, the conformational transition from Silk I to Silk II occurs. In this case, the peaks position arising from the alanine and glycine residues is also in agreement between the two samples.

### Industrial Applicability

According to our invention, Silk-like materials may have different functions by modifying some constituents in the copolymer. Moreover, the precursor presents the unique properties of natural silk when spun into fibers. Since the copolymer is a protein, it is biodegradable and therefore, our method of producing this silk-like material is ecological.

## Claims

1. The silk-like material precursor **characterized** as a copolymer represented by the general formula -[(GA¹)ⱼ-((GA²)ₖ-G-Y-(GA³)ₗ)ₘ]ₙ-, containing repeated β-turn type II structures in the module (GA¹)ⱼ and having intramolecular hydrogen bonds formed successively along its molecular axis. In this formula, G represents glycine, A¹ represents alanine, but it is also possible to replace every third A¹ with serine, A² and A³ both represent alanine, but these residues may be partly replaced with valine. Y represents an amino acid with an asymmetric carbon atom giving water solubility to the precursor. The j is an integer of at least 6, k and l are both integers of 0 to 5, m is an integer of 1 to 7 and n is an integer of at least 10.

2. In the precursors of silk-like material as described in the claim 1, Y in the formula is L-tyrosine and this should be contained in a proportion lower than 20 mole %.

3. The silk-like material precursors as described in claims 1 or 2, wherein Y in the formula is L-tyrosine.

4. A method for producing the silk-like material precursors with the following characteristics. The copolymers were designed according to the general formula defined in claim 1. Then, at first, the oligonucleotides with the desired sequence is chemically synthesized and the repetition units in the formula are prepared using these oligonucleotides. Then it is incorporated into a cloning vector in order to do the polymerization to an appropriate extent. After incorporating the repetition units or its polymers into an expression vector, the vector is transferred into *Escherichia coli* in order to express the precursor.

5. The silk-like materials which were **characterized by** spinning of the silk-like material precursors described in any of claims 1 to 3 lead to a transition from the intra- and inter-molecular hydrogen bonding formations along the chain to all inter-molecular hydrogen bonding formations.

6. The silk-like materials as described in claim 5, wherein the spinning is performed under a water-containing condition.

7. A method of producing a silk-like materials with Silk II structure. This is **characterized by** spinning from the solution containing the silk-like material precursors as described in claim 1, where the precursor with intra-molecular hydrogen bonds changes to the sample with intermolecular hydrogen bonds by spinning and then drawing, if necessary.

8. A method of producing silk-like materials as described in claim 7, wherein the solution of the silk materials is prepared as follows. The dried films with amorphous or Silk I-structure are prepared from the aqueous solution of the silk-like material precursors and then dissolved in hexafluoroisopropanol at temperature of 15°C to 30°C.


SEQUENCE LISTING
<110> TOKYO UNIVERSITY OF AGRICULTUR & TECHNOLOGY
<120> Silklike Material Precursor, Silklike Material and the Process for producing
thereof
<160> 10
<210> 1
<211> 11
<212> PRT
<213> Artificial Sequence
<400> 1
Gly Ala Gly Ala Gly Ser Gly Ala Gly Ala Gly
1               5                   10
<210> 2
<211> 11
<212> PRT
<213> Artificial Sequence
<400> 2
Gly Ala Gly Ala Gly Tyr Gly Ala Gly Ala Gly
1               5                   10
<210> 3
<211> 11
<212> PRT
<213> Artificial Sequence
<400> 3
Gly Ala Gly Val Gly Tyr Gly Ala Gly Ala Gly
1               5                   10
<210> 4
<211> 30
<212> PRT
<213> Artificial Sequence
<400> 4
Thr Gly Phe Asp Ser Glu Ser Ser Trp Ala Tyr Glu Tyr Gly Ser
1               5                   10                  15
Tyr Gly Gly Asn Ala Val Tyr Pro Gly Phe Gly Ser Ser Gly Thr
                20                  25                  30
<210> 5

<211> 24
<212> PRT
<213> Artificial Sequence
<400> 5
Ala Gly Ala Gly Ala Gly Ala Gly Ala Gly Ala Gly Tyr Gly Ala
1               5                   10                  15
Gly Ala Gly Ala Gly Ala Gly Ala Gly
                20
<210> 6
<211> 30
<212> PRT
<213> Artificial Sequence
<400> 6
Ala Gly Ala Gly Ala Gly Ala Gly Ala Gly Ala Gly Ala Gly Tyr
1               5                   10                  15
Gly Ala Gly Ala Gly Ala Gly Ala Gly Ala Gly Ala Gly Ala Gly
                20                  25                  30
<210> 7
<211> 30
<212> PRT
<213> Artificial Sequence
<400> 7
Ala Gly Ala Gly Ala Gly Ala Gly Ala Gly Ala Gly Ala Gly Ala
1               5                   10                  15
Gly Ala Gly Ala Gly Ala Gly Ala Gly Ala Gly Ala Gly Ala Gly
                20                  25                  30
<210> 8
<211> 30
<212> PRT
<213> Artificial Sequence
<400> 8
Ala Gly Ala Gly Ala Gly Tyr Gly Ala Gly Ala Gly Ala Gly Tyr
1               5                   10                  15
Gly Ala Gly Ala Gly Ala Gly Tyr Gly Ala Gly Ala Gly Ala Gly
                20                  25                  30
<210> 9

<211> 30
<212> PRT
<213> Artificial Sequence
<400> 9
Ala Gly Ala Gly Ala Gly Tyr Gly Ala Gly Ala Gly Val Gly Tyr
1               5                   10                  15
Gly Ala Gly Ala Gly Ala Gly Tyr Gly Ala Gly Ala Gly Ala Gly
                20                  25                  30
<210> 10
<211> 30
<212> PRT
<213> Artificial Sequence
<400> 10
Ala Gly Ala Gly Tyr Gly Ala Gly Ala Gly Tyr Gly Ala Gly
1               5                   10
Ala Gly Tyr Gly Ala Gly Ala Gly Tyr Gly Ala Gly Ala Gly
15                  20                  25
Tyr Gly
    30
<210> 11
<211> 30
<212> PRT
<213> Artificial Sequence
<222> (14)
<223> Xaa represents [1-13C].
<222> (15)
<223> Xaa represents [1-13C]A.
<400> 11
Ala Gly Ala Gly Ala Gly Ala Gly Ala Gly Ala Gly Ala Xaa Xaa
1               5                   10                  15
Gly Ala Gly Ala Gly Ala Gly Ala Gly Ala Gly Ala Gly Ala Gly
                20                  25                  30

<210> 12
<211> 30
<212> PRT

<213> Artificial Sequence
<222> (15)
<223> Xaa represents [1-13C]A.
<222> (16)
<223> Xaa represents [1-13C]G.
<400> 12
Ala Gly Ala Gly Ala Gly Ala Gly Ala Gly Ala Gly Ala Gly Xaa
1               5                   10                  15
Xaa Ala Gly Ala Gly Ala Gly Ala Gly Ala Gly Ala Gly Ala Gly
                20                  25                  30
<210> 13
<211> 30
<212> PRT
<213> Artificial Sequence
<222> (14)
<223> Xaa represents [1-13C]G.
<222> (16)
<223> Xaa represents [1-13C]G.
<400> 13
Ala Gly Ala Gly Ala Gly Ala Gly Ala Gly Ala Gly Ala Xaa Ala
1               5                   10                  15
Xaa Ala Gly Ala Gly Ala Gly Ala Gly Ala Gly Ala Gly Ala Gly
                20                  25                  30

<210> 14
<211> 30
<212> PRT
<213> Artificial Sequence
<222> (14)
<223> Xaa represents [1-13C]G.
<222> (16)
<223> Xaa represents [15N]G.
<400> 14
Ala Gly Ala Gly Ala Gly Ala Gly Ala Gly Ala Gly Ala Gly Ala
1               5                   10                  15
Gly Ala Gly Ala Gly Ala Gly Ala Gly Ala Gly Ala Gly Ala Gly

                20                  25                  30
<210> 15
<211> 30
<212> PRT
<213> Artificial Sequence
<222> (15)
<223> Xaa represents [1-13C]A.
<222> (17)
<223> Xaa represents [15N]A.
<400> 15
Ala Gly Ala Gly Ala Gly Ala Gly Ala Gly Ala Gly Ala Gly Xaa
1               5                   10                  15
Gly Xaa Gly Ala Gly Ala Gly Ala Gly Ala Gly Ala Gly Ala Gly
                20                  25                  30
<210> 16
<211> 30
<212> PRT
<213> Artificial Sequence
<222> (14)
<223> Xaa represents [1-13C]G.
<222> (17)
<223> Xaa represents [15N]A.
<400> 16
Ala Gly Ala Gly Ala Gly Ala Gly Ala Gly Ala Gly Ala Xaa Ala
1               5                   10                  15
Gly Xaa Gly Ala Gly Ala Gly Ala Gly Ala Gly Ala Gly Ala Gly